# FASCICULE DE BREVET EUROPEEN

(11) **EP 1 470 800 B1**
(45) Date de publication et mention de la délivrance du brevet: **21.02.2018**
(21) Numéro de dépôt: 04290993.7
(22) Date de dépôt: 14.04.2004
(51) Int. Cl.: A61F 2/38

(54) **Plot modulaire pour prothèse du genou postéro-stabilisée**
Modularer Zapfen für eine in der posterioren Richtung stabilisierten Knieprothese.
Modular guide post for knee prothesis with posterior stabilisation.

(30) Priorité: 24.04.2003 FR 0305059
(43) Date de publication de la demande: 27.10.2004
(73) Titulaire: Aesculap S.A.S., 52000 Chaumont (FR)
(72) Inventeur: Plumet, Melle Sylvie, 52000 Chaumont (FR); Moussa, Said, 52000 Chamarandes-Choignes (FR)
(74) Mandataire: Eidelsberg, Olivier Nathan

(56) Documents cités:
- EP-A- 0 627 203
- EP-A- 0 923 916
- EP-A- 0 970 667
- EP-A- 1 108 403
- WO-A-02/34156
- FR-A- 2 760 352

## Description

La présente invention se rapporte à un plot tibial d'une prothèse du genou, dite postéro-stabilisée, notamment d'une prothèse du genou d'essai, ainsi qu'à une prothèse du genou comportant un plot de ce genre, notamment une prothèse d'essai.

Ce type de prothèse comporte un implant tibial et un implant fémoral. L'implant tibial comporte un plateau tibial sur lequel repose un insert tibial (appelé également ménisque), généralement en polyéthylène. L'insert tibial comporte d'une part une base sur la partie supérieure de laquelle sont formées des surfaces supérieures, en général concaves, destinées à coopérer avec des surfaces extérieure de condyles, généralement convexes, de l'implant fémoral et d'autre part un plot tibial faisant saillie de la base, notamment perpendiculairement, et destiné à coopérer avec un plot fémoral associé. Le plot fémoral est sensiblement transversal en s'étendant d'un condyle à l'autre de l'implant fémoral et bute contre le plot tibial pour empêcher un glissement antéro postérieur de l'implant fémoral sur un domaine de flexion du genou, en particulier à partir d'une flexion de 30°.

En fonction de l'anatomie du fémur et du tibia sur lesquels doit être installée la prothèse, en particulier leur orientation mutuelle et le volume de résection osseuse à effectuer, il est nécessaire de sélectionner un insert tibial parmi plusieurs tailles d'insert tibial possibles.

Lorsqu'un chirurgien installe ce type de prothèse, il doit vérifier d'une part la taille de l'implant et aussi le bon positionnement du plot tibial. Il doit donc avoir à disposition l'ensemble des différents inserts tibiaux possibles, sous la forme de prothèses d'essai, ce qui implique la teneur de stocks importants. En outre, il est nécessaire d'avoir une prothèse d'essai stable et résistante aux efforts comme une prothèse réelle, afin de pouvoir vérifier que la taille choisie est adaptée aux besoins.

EP 1108403 décrit une prothèse suivant le préambule de la revendication 1.

La présente invention vise une prothèse totale du genou postéro-stabilisée, notamment une prothèse d'essai suivant la revendication 1.

Ainsi, le plot, après avoir traversé la base, peut également pénétrer dans un alésage formé dans le plateau tibial, voir la quille d'ancrage dans le tibia. Ainsi, il est possible d'avoir à disposition plusieurs bases de tailles différentes, notamment d'épaisseurs différentes, ayant toutes le même alésage de traversée pour recevoir un même ergot. On obtient avec un seul plot des inserts de tailles différentes pour des prothèses d'essai qui sont cependant très robustes vis-à-vis des efforts et contraintes et donc qui permettent de réaliser de véritables essais. En particulier, l'ergot qui traverse la base maintient bien en position le plot fixe par rapport à la base, sans que celui ci puisse basculer en raison d'un impact de la partie fémorale sur le plot.

Suivant un perfectionnement de l'invention, le plot tibial comporte un socle de base sensiblement plat et de forme non circulaire, en particulier sensiblement rectangulaire ou carrée, dont fait saillie l'ergot, tandis que l'insert tibial comporte un évidement à sa surface supérieure de dimension et forme complémentaire du socle afin de le recevoir en en empêchant la rotation par rapport à l'axe de l'ergot.

Suivant un mode de réalisation préféré, l'ergot est de forme cylindrique, notamment circulaire, et l'alésage est de forme complémentaire de l'ergot.

Suivant un mode de réalisation préféré, un joint torique de coincement est enfilé sur l'ergot, afin de permettre un blocage de l'ergot dans l'alésage.

La présente invention vise également un ensemble d'inserts tibiaux, notamment pour prothèse d'essai, constitué d'une pluralité de bases ayant des tailles différentes et d'un plot tibial suivant l'invention, les bases ayant toutes le même alésage, voir le même évidement.

On décrit maintenant une prothèse d'essai du genou suivant un mode de réalisation de l'invention, uniquement à titre d'exemple, en se reportant aux dessins, dans lesquels :
la figure 1 représente un insert tibial monté sur une partie tibial d'une prothèse de genou, à l'état non assemblé, et
la figure 2 représenté la partie tibiale de la figure 1, à l'état assemblé.

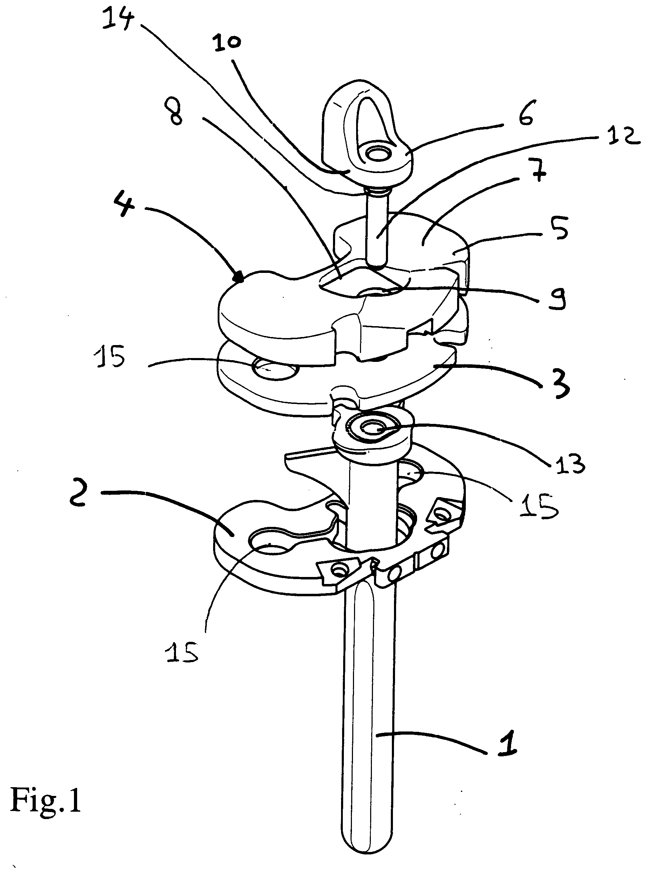

A la figure 1, on peut voir une partie tibiale d'une prothèse d'essai de genou, comportant une quille 1 d'ancrage dans le canal médullaire du tibia, un plateau tibial 2, une entretoise 3 et un insert 4, en polymère.

L'insert 4 comporte une base 5 et un plot 6. La surface supérieure de la base 5 comporte des surfaces concaves 7 de contact avec des condyles d'une partie fémorale.

La base 5 comporte un évidement 8 de fond sensiblement carré dans lequel est formé un alésage 9 de traversée de la face supérieure à la face inférieure de la base. L'alésage 9 est de section circulaire. La section transversale de l'alésage est plus petite que le fond de l'évidement 8.

Deux plots de fixation droit et gauche font saillies de la face inférieure de la base 5, et pénètrent dans des trous 15 droit et gauche complémentaires formés dans le plateau tibial 2 et l'entretoise 3, pour permettre une fixation de l'insert 4 tibial par rapport au plateau 2. En particulier, une rotation de l'insert 4 par rapport au plateau 2 par rapport à l'axe de la quille 1 est empêché par ces plots et trous 15 de forme complémentaires. Bien évidemment, d'autres moyens de fixation de l'insert au plateau tibial sont possibles.

Le plot 6 comporte un socle 10 de forme complémentaire de l'évidement dont est issu vers le haut une sorte de bossage 11 destiné à venir en contact avec un plot fémoral s'étendant entre les deux condyles de la partie fémorale, dans la direction médio-latérale.

Le socle 10 et l'évidemment 8 n'ont pas une forme circulaire, de sorte que le socle ne peut pas tourner à l'intérieur de l'évidemment par rapport à l'axe de la quille 1.

De la face plane inférieure du plot 6, il est issu un ergot 12 cylindrique circulaire oblong de forme complémentaire de celle de l'alésage 9.

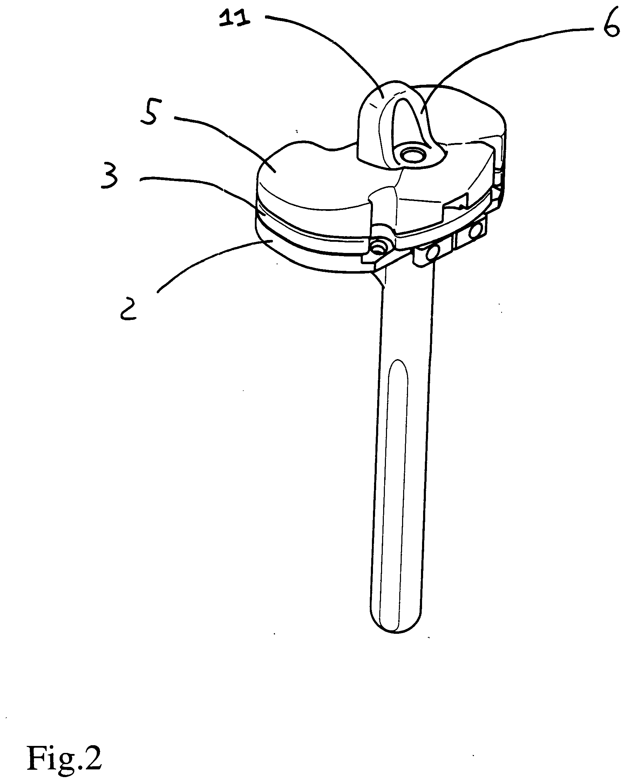

En position assemblée de la prothèse (figure 2), le plot pénètre dans l'alésage avec ajustement serré et le socle 10 est posé sur le fond de l'évidement 8, notamment avec encliquetage.

La longueur de l'ergot est supérieure à l'épaisseur de la base au niveau de l'alésage de traversée, de sorte que l'ergot "ressort" de l'autre côté.

La longueur de l'ergot est supérieure à la somme de l'épaisseur de la base, de l'épaisseur de l'entretoise éventuelle et de l'épaisseur du plateau tibial, de manière à pénétrer dans un évidement 13 de quille formé dans la quille de la prothèse.

Dans le cas par exemple où il existe quatre épaisseurs différentes possibles pour la base, l'ergot est dimensionné en longueur en prenant en compte l'épaisseur la plus grande.

Un joint torique 14 est emmanché autour de l'ergot, de sorte qu'une fois introduit dans l'alésage, le joint aura tendance à l'y bloquer.

Le fait que l'ergot traverse la base 5 et vient pénétrer dans la quille permet d'obtenir une prothèse d'essai particulièrement rigide et résistance aux efforts, de sorte que les essais que l'on réalise avec peuvent être considérés comme aussi fiables qu'avec une prothèse d'essai à plot fixe. L'avantage étant bien entendu que cette fiabilité de l'essai peut être obtenue sans avoir besoin d'avoir à disposition toute la gamme en dimension des parties tibiales.

## Revendications

1. Prothèse totale du genou postéro-stabilisée comportant une partie tibiale ayant un plateau tibial, une partie fémorale et un insert tibial interposé entre les parties fémorale et tibiale, fixé par des moyens de fixation au plateau tibial et comportant une base (5) sur la partie supérieure de laquelle sont formées une ou des surfaces concaves de contact avec un ou des condyles d'une partie fémorale, et un plot (6) tibial faisant saillie, notamment perpendiculairement de la base, le plot tibial étant monté amovible par rapport à la base, le plot tibial comportant un ergot (12) issu de sa surface inférieure de longueur plus grande que l'épaisseur de la base et la base comporte un alésage (9) de traversée dans laquelle passe le plot tibial pour faire saillie hors de la surface inférieure de la base, opposée à la surface supérieure, **caractérisée en ce que** l'agencement est réalisé de sorte que le plot ne peut pas pivoter par rapport à la base par rapport à l'axe de l'ergot.

2. Prothèse suivant la revendication 1, **caractérisée en ce que** le plot tibial comporte un socle de base sensiblement plat et de forme non circulaire, en particulier sensiblement rectangulaire ou carrée, dont fait saillie l'ergot, tandis que l'insert tibial comporte un évidement à sa surface supérieure de dimension et forme complémentaire du socle afin de le recevoir en en empêchant la rotation par rapport à l'axe de l'ergot.

3. Prothèse suivant la revendication 1 ou 2, **caractérisée en ce que** l'ergot est de forme cylindrique, notamment circulaire, et l'alésage est de forme complémentaire de l'ergot.

4. Prothèse suivant la revendication 1, 2 ou 3, **caractérisée en ce qu'**un joint torique de coincement est enfilé sur l'ergot, afin de permettre un blocage de l'ergot dans l'alésage.

5. Prothèse suivant l'une des revendications précédentes, **caractérisée en ce qu'**il s'agit d'une prothèse d'essai.

6. Ensemble d'inserts tibiaux pour prothèse d'essai suivant la revendication 5, constitué
- d'une pluralité de bases ayant des tailles différentes et chaque base (5) ayant sur sa partie supérieure une ou des surfaces concaves de contact avec un ou des condyles d'une partie fémorale; et
- d'un plot (6) tibial comportant un ergot (12) issu de sa surface inférieure de longueur plus grande que l'épaisseur de chaque base et chaque base comporte un alésage (9) de traversée dans laquelle peut passer le plot tibial pour faire saillie hors de la surface inférieure de chaque base, opposée à la surface supérieure, l'agencement étant réalisé de sorte que le plot ne peut pas pivoter par rapport à chaque base par rapport à l'axe de l'ergot.

7. Ensemble d'inserts tibiaux suivant la revendication 6, **caractérisé en ce que** le plot tibial comporte un socle de base sensiblement plat et de forme non circulaire, en particulier sensiblement rectangulaire ou carrée, dont fait saillie l'ergot, tandis que chaque base comporte un évidement à sa surface supérieure de dimension et forme complémentaire du socle afin de le recevoir en en empêchant la rotation par rapport à l'axe de l'ergot.

8. Ensemble d'inserts tibiaux suivant la revendication 6 ou 7, **caractérisé en ce que** l'ergot est de forme cylindrique, notamment circulaire, et l'alésage est de forme complémentaire de l'ergot.

## Patentansprüche

1. Totalprothese des Knies, die in posteriorer Richtung stabilisiert ist, umfassend einen Tibialteil mit einem Tibialplateau, einen Femoralteil und einen Tibialeinsatz, der zwischen dem Femoral- und Tibialteil zwischengefügt ist, durch Befestigungsmittel am Tibialplateau befestigt ist und eine Basis (5) umfasst, an deren oberen Teil eine oder mehrere konkave Kontaktflächen mit einem oder mehreren Kondylen eines Femoralteils und ein Tibialzapfen (6), der insbesondere senkrecht auf die Basis herausragt, ausgebildet sind, wobei der Tibialzapfen abnehmbar in Bezug zur Basis montiert ist, wobei der Tibialzapfen einen Haken (12) umfasst, der aus seiner Unterseite mit einer größeren Länge als die Dicke der Basis herausragt, und wobei die Basis eine Durchgangsöffnung (9) umfasst, durch die der Tibialzapfen hindurchgeht, um aus der Unterseite der Basis gegenüber der Oberseite herauszuragen, **dadurch gekennzeichnet, dass** die Anordnung derart ist, dass der Zapfen nicht in Bezug zur Basis in Bezug zur Achse des Hakens schwenken kann.

2. Prothese nach Anspruch 1, **dadurch gekennzeichnet, dass** der Tibialzapfen einen im Wesentlichen flachen Basissockel von nicht kreisförmiger Form, insbesondere im Wesentlichen rechteckiger oder quadratischer Form, umfasst, aus dem der Haken herausragt, während der Tibialeinsatz eine Ausnehmung auf seiner Oberseite mit zum Sockel komplementärer Abmessung und Form umfasst, um diesen aufzunehmen, wobei dessen Drehung in Bezug zur Achse des Hakens verhindert wird.

3. Prothese nach Anspruch 1 oder 2, **dadurch gekennzeichnet, dass** der Haken von zylindrischer Form, insbesondere kreisförmiger Form, ist, und die Öffnung von zum Haken komplementärer Form ist.

4. Prothese nach Anspruch 1, 2 oder 3, **dadurch gekennzeichnet, dass** ein Dichtungsklemmring auf den Haken aufgeschoben ist, um eine Feststellung des Hakens in der Öffnung zu ermöglichen.

5. Prothese nach einem der vorhergehenden Ansprüche, **dadurch gekennzeichnet, dass** es sich um eine Testprothese handelt.

6. Gesamtheit von Tibialeinsätzen für Testprothesen nach Anspruch 5, die gebildet ist von:
- einer Vielzahl von Basisflächen, die unterschiedliche Größe haben, und wobei jede Basis (5) in ihrem oberen Teil eine oder mehrere konkave Kontaktflächen mit einem oder mehreren Kondylen eines Femoralteils hat; und
- einem Tibialzapfen (6), umfassend einen Haken (12), der aus seiner Unterseite mit einer größeren Länge als die Dicke jeder Basis herausragt, und wobei jede Basis eine Durchgangsöffnung (9) umfasst, durch die der Tibialzapfen hindurchgehen kann, um aus der Unterseite der Basis gegenüber der Oberseite herauszuragen, wobei die Anordnung derart ist, dass der Zapfen nicht in Bezug zu jeder Basis in Bezug zur Achse des Hakens schwenken kann.

7. Gesamtheit von Tibialeinsätzen nach Anspruch 6, **dadurch gekennzeichnet, dass** der Tibialzapfen einen im Wesentlichen flachen Basissockel von nicht kreisförmiger Form, insbesondere im Wesentlichen rechteckiger oder quadratischer Form, umfasst, aus dem der Haken herausragt, während jede Basis eine Ausnehmung auf ihrer Oberseite mit zum Sockel komplementärer Abmessung und Form umfasst, um diesen aufzunehmen, wobei dessen Drehung in Bezug zur Achse des Hakens verhindert wird.

8. Gesamtheit von Tibialeinsätzen nach Anspruch 6 oder 7, **dadurch gekennzeichnet, dass** der Haken von zylindrischer Form, insbesondere kreisförmiger Form, ist, und die Öffnung von zum Haken komplementärer Form ist.

## Claims

1. A posterior stabilised complete knee prosthesis, comprising
a tibial part which has a tibial disc, a femoral part and a tibial insert interposed between the femoral and tibial parts, fitted by fixing methods to the tibial disc and made up of a base (5) on the upper part from which one or several concave contact surfaces are formed with one or several condyles of a femoral part, and
a tibial stud (6) which protrudes, notably at right angles to the base, the tibial stud being detachable from the base, wherein
the tibial stud comprises a pin (12) coming from its lower surface which is greater in length than the thickness of the base and
the base comprises a cross bore (9) into which the tibial part goes so that it projects out of the lower surface of the base, opposite the upper surface, **characterized in that** the construction is such that the stud cannot rotate in relation to the base around the pin axis.

2. A prosthesis according to claim 1, **characterized in that** the tibial stud is made up of a noticeably flat, non-circular plinth, in particular noticeably rectangular or square, from which the pin protrudes, while the tibial insert comprises a cavity in its upper surface of a size and shape complementary to the plinth in order to prevent rotation around the pin axis.

3. A prosthesis according to claim 1 or 2, **characterized in that** the pin is cylindrical in shape, noticeably circular, and the bore is of a shape complementary to the pin.

4. A prosthesis according to claim 1, 2 or 3, **characterized in that** an O-ring wedge is threaded onto the pin, in order to facilitate it being locked in the bore.

5. A prosthesis according to any one of the preceding claims, **characterized in that** it is in the form of a trial prosthesis.

6. A group of tibial inserts for trial prosthesis in accordance with claim 5, comprising
a plurality of bases of differing sizes, each of said bases (5) having on its upper part one or more concave contact surfaces with one or more condyles of a femoral part;
a tibial stud (6) having a pin (12) coming from it's lower surface which is greater in length than the thickness of each base and
each base has a cross bore (9) through which the tibial stud can pass in order to protrude out of the lower surface of each base, opposite the higher surface, being constructed in such a way that the stud cannot rotate in relation to each base and round the axis of the pin.

7. A group of tibial inserts according to claim 6, **characterized in that** the tibial stud comprises a base plinth noticeably flat and non-circular in shape, in particular substantially rectangular or square, with the pin projecting from it, while each base comprises a cavity in its upper surface of a dimension and shape complementary to the plinth in order to prevent rotation round the axis of the pin.

8. A group of tibial inserts according to claim 6 or 7, **characterized in that** the pin is cylindrical in shape, noticeably circular, and the bore is of a shape complementary to the pin.
